(19) Europäisches Patentamt
European Patent Office
Office européen des brevets

(11) Veröffentlichungsnummer: **0 471 201 A1**

(12) **EUROPÄISCHE PATENTANMELDUNG**

(21) Anmeldenummer: **91112046.7**

(22) Anmeldetag: **18.07.91**

(51) Int. Cl.[5]: **C07D 413/12**, C09K 19/34, G02F 1/13, C07D 417/12, C07D 263/20

(30) Priorität: **21.07.90 DE 4023216**
**24.07.90 DE 4023493**

(43) Veröffentlichungstag der Anmeldung:
**19.02.92 Patentblatt 92/08**

(84) Benannte Vertragsstaaten:
**CH DE GB LI**

(71) Anmelder: **HOECHST AKTIENGESELLSCHAFT**
**Postfach 80 03 20**
**W-6230 Frankfurt am Main 80(DE)**

(72) Erfinder: **Scherowsky, Günter, Prof. Dr.**
**Winklerstrasse 18 b**
**W-1000 Berlin(DE)**
Erfinder: **Sefkow, Michael, Dr.**
**Eichenallee 69**
**W-1000 Berlin(DE)**
Erfinder: **Illian, Gerd, Dr.**
**Rauenthaler Weg 32**
**W-6000 Frankfurt am Main(DE)**
Erfinder: **Wingen, Rainer, Dr.**
**Brunnenstrasse 1**
**W-6234 Hattersheim am Main(DE)**

(54) **Neue Oxazolidinon-Derivate und ihre Verwendung als Dotierstoffe in Flüssigkristallmischungen.**

(57) Chirale Oxazolidinone gemäß den allgemeinen Formeln (I) oder (II)

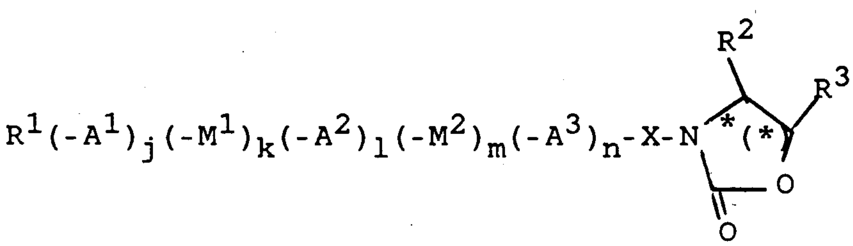

(I)

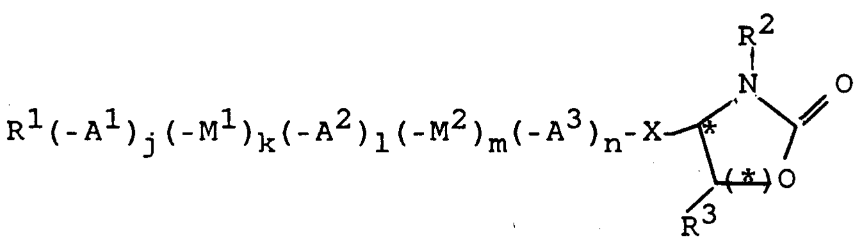

(II)

wobei die Symbole folgende Bedeutung haben:

$R^1$ z.B. geradkettiger oder verzweigter Alkylrest mit 1 bis 16 C-Atomen oder

$R^2$, $R^3$ z.B. -H, $-CH_3$, oder Phenyl

j, l und n null, 1 oder 2,

k und m null oder 1

EP 0 471 201 A1

$A^1$, $A^2$, $A^3$ z.B. Phenyl, Cyclohexyl,
$M^1$, $M^2$ z.B. COO oder $CH_2O$ und
X z.B. $CH_2$ oder COO
eigenen sich als Dotierstoffe in Flüssigkristallmischungen. Sie führen zu flüssigkristallinen ferroelektrischen Phasen mit kurzen Schaltzeiten. Ein weiterer Vorteil liegt darin, daß die Oxazolidinone eine Helix mit sehr kleiner Ganghöhe induzieren, so daß sie auch zur Helixkompensation in LC-Mischungen geeignet sind.

Flüssigkristalle haben insbesondere im letzten Jahrzehnt Eingang in verschiedene technische Gebiete gefunden, in denen elektrooptische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Ausrichtungseffekten in den nematischen, cholesterischen und/oder smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, zu lang. Dieser Nachteil macht sich insbesondere dann bemerkbar, wenn eine große Anzahl von Bildpunkten angesteuert werden muß. Die Herstellungskosten von Geräten, die größere Bildschirmflächen enthalten, wie z.B. von Videogeräten sind dann im allgemeinen zu hoch.

Neben den nematischen und cholesterischen Flüssigkristallen haben seit einigen wenigen Jahren in zunehmendem Maß auch optisch aktive smektische Flüssigkristall-Phasen an Bedeutung gewonnen.

Clark und Lagerwall konnten zeigen, daß die Verwendung ferroelektrischer Flüssigkristallsysteme in sehr dünnen Zellen zu optoelektrischen Schalt- oder Anzeigeelementen führt, die im Vergleich zu den herkömmlichen TN ("twisted nematic")-Zellen um bis zu einem Faktor 1000 schnellere Schaltzeiten haben (vgl. z.B. Lagerwall et al. "Ferroelectric Liquid Crystals for Displays", SID Symposium, October Meeting 1985, San Diego, Ca., USA). Aufgrund dieser und anderer günstiger Eigenschaften, z.B. der bistabilen Schaltmöglichkeit und des nahezu blickwinkelunabhängigen Kontrasts, sind FLC's grundsätzlich für die obengenannten Anwendungsgebiete, z.B. über eine Matrixansteuerung, gut geeignet.

Für elektrooptische Schalt- und Anzeigeelemente benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A^*$ und $S_C^*$ -Phase läßt sich erreichen, wenn die Phasenfolge der Flüssigkristallmischung mit abnehmender Temperatur lautet:

Isotrop $\rightarrow$ N* $\rightarrow$ $S_A^*$ $\rightarrow$ $S_C^*$

Voraussetzung ist, daß der Pitch (Ganghöhe der Helix) in der N*-Phase sehr groß (größer 10 $\mu$m) oder noch besser völlig kompensiert ist (siehe z.B. T. Matsumoto et al., p. 468-470, Proc. of the 6th Int. Display Research Conf., Japan Display, Sept. 30 - Okt. 2, 1986, Tokyo, Japan; M. Murakami et al., ibid. S.344 - S.347). Dies erreicht man, indem man zu der chiralen Flüssigkristallmischung, die in der N*-Phase z.B. eine linksdrehende Helix aufweist, einen weiteren optisch aktiven Dotierstoff, der eine rechtsdrehende Helix induziert, in solchen Mengen hinzugibt, daß die Helix gerade kompensiert wird.

Es wurde nun gefunden, daß optisch aktive Oxazolidinone als Dotierstoffe in geneigt smektischen Flüssigkristallphasen schon bei geringen Zumischmengen zu einer starken Verdrillung in der cholesterischen Phase führen.

Diese in der N*-Phase induzierte Helix kann in Mischungen vorteilhaft zur gezielten Kompensation der Ganghöhe verwendet werden. Besonders vorteilhaft ist dabei, daß die erfindungsgemäßen Dotierstoffe aufgrund ihres starken Verdrillungsvermögens schon in geringen Zusatzmengen den Pitch eines anderen Dotierstoffes kompensieren.

Gegenstand der Erfindung ist daher die Verwendung von chiralen bzw. optisch aktiven Oxazolidinonen als Dotierstoffe in Flüssigkristallmischungen. Gegenstand der Erfindung sind weiterhin Flüssigkristallsysteme, die chirale bzw. optisch aktive Oxazolidinone enthalten, sowie neue chirale Oxazolidinone (sowohl als optisch aktive Verbindungen wie auch als racemische Gemische). Die gemäß der Erfindung einzusetzenden Oxazolidinone entsprechen den allgemeinen Formeln (I) oder (II)

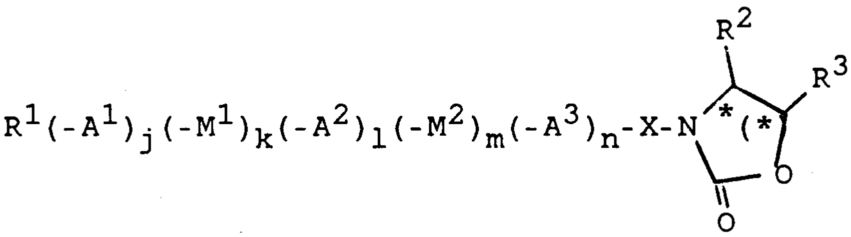

(I)

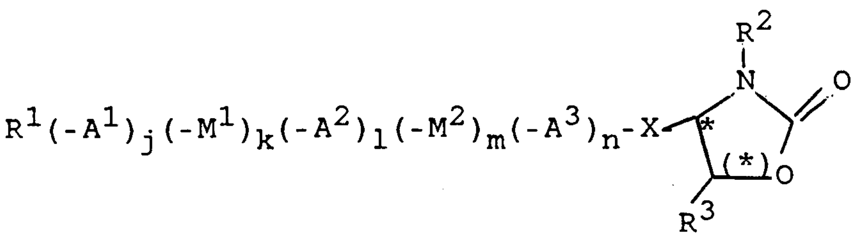

(II)

in denen die Symbole und Indices folgende Bedeutung haben:

- * chirales Zentrum,
- (*) gegebenenfalls chirales Zentrum,
- $R^1$ ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, eine oder mehrere nicht benachbarte $-CH_2$-Gruppen durch -O-, -S-, -CO-, -O-CO- und/oder -CO-O- ersetzt sein können, und wobei ein oder mehrere H durch F, Cl, Br oder CN ersetzt sein können,

$-R^2$, $-R^3$ unabhängig voneinander -H, ein Alkyl mit 1 bis 12 C-Atomen, bei dem eine $-CH_2$-Gruppe auch durch -O- oder -S- ersetzt sein kann, und bei dem die terminale $CH_3$-Gruppe auch durch $CH_2OR^4$, $CH_2SR^4$, $CH_2COOR^4$ oder $CH_2CONH_2$ ersetzt sein kann, wobei $R^4$ Alkyl mit 1 bis 10 C-Atomen ist, -Phenyl oder -Cyclohexyl, die auch durch $-OR^4$, -F oder -J substituiert sein können; (vorzugsweise bedeuten

$-R^2$ und $-R^3$ -H, $-CH_3$,

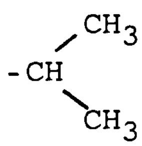 , 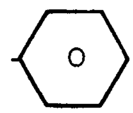 , 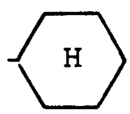 ,

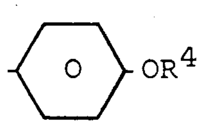 ;

j und l null, 1 oder 2,

k und m null oder 1,

n null, 1 oder 2,

mit folgender Maßgabe: wenn j und/oder l = null sind, ist k = null; wenn n = null ist, ist m = null; die Summe j + l + n ist minimal 1 und maximal 3,

$-A^1$, $-A^2$

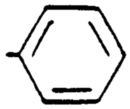 , 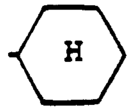 , 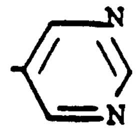 , 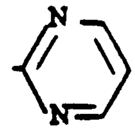 , 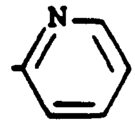 ,

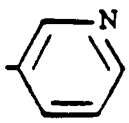 , 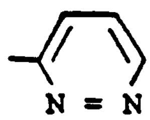 , 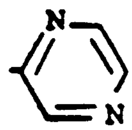 , 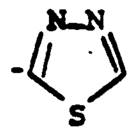 , 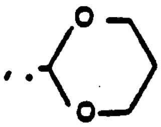 ,

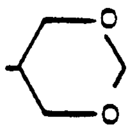 , 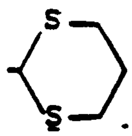 , 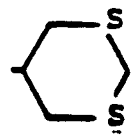 , 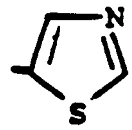 , 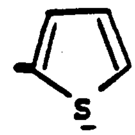 ,

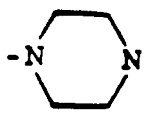 , 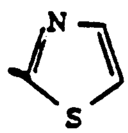

$-A^3$

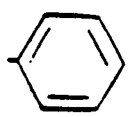 , 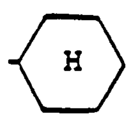 , 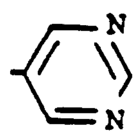 , 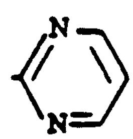 , 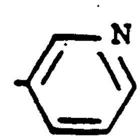 ,

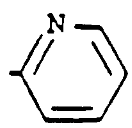 , 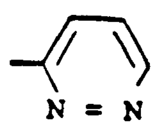

$-M^1$, $-M^2$ -CO-O, -O-CO, $-CH_2CH_2$, -CH = CH, $-CH_2O$, $-OCH_2$, -X- $-O-CH_2$, $-CH_2-$,

$$-\overset{O}{\overset{\|}{C}}-O-,\quad -O-\overset{O}{\overset{\|}{C}}-,$$

$-CH_2-O-$, $-C_2H_4-$.

In einer bevorzugten Ausführungsform haben die Symbole in der allgemeinen Formel (I) die folgende Bedeutung:

$R^1$ ein geradkettiger oder verzweigter Alkyl- oder Alkenylrest mit 4 bis 14 C-Atomen, der ein asymmetrisches C-Atom enthalten kann, oder wobei eine $-CH_2$-Gruppe durch -O-, -CO- oder -CO-O- ersetzt sein kann, oder wobei ein oder mehrere H durch F ersetzt sein können

j, k, l, m null oder 1.

In einer weiteren bevorzugten Ausführungsform werden Oxazolidinone der allgemeinen Formel (I) eingesetzt, wobei

$R^1$ ein geradkettiger oder verzweigter Alkyl- oder Alkenylrest mit 6 bis 12 C-Atomen ist, der ein asymmetrisches C-Atom enthalten kann, und wobei eine $CH_2$-Gruppe durch -O-, -CO-, oder -COO- ersetzt sein kann, und

die Gruppierung $(-A^1)_j(-M^1)_k(-A^2)_l(-M^2)_m(-A^3)_n$-folgende Bedeutung hat:

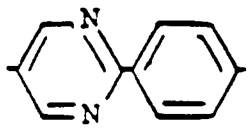 , 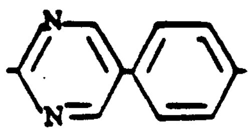 , 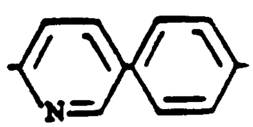 ,

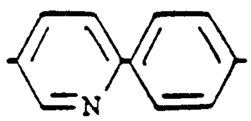 ,

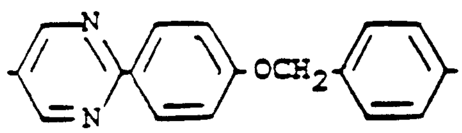 , 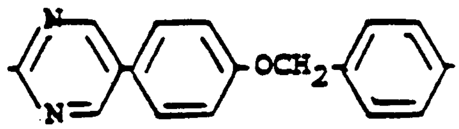 ,

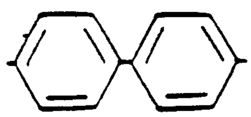 , 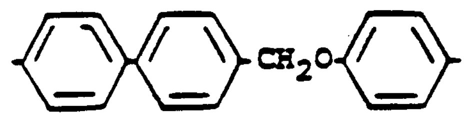 ,

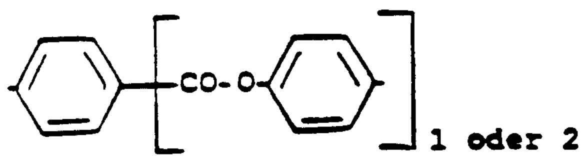 , 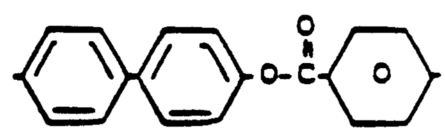

Ebenfalls bevorzugt werden solche Oxazolidinone der allgemeinen Formel (I) oder (II), bei denen M gleich COO, OCO, $CH_2O$ oder $OCH_2$ ist, und X eine Gruppe $CH_2$, $OCH_2$ oder $CH_2O$ bedeutet.

Prinzipiell sind jedoch auch Oxazolidinone Gegenstand der Erfindung, bei denen die mesogene Gruppierung $(R^1(-A^1)\ (-M^1)_k(-A^2)_l(-M^2)_m(-A^3)_n-)$ in 5-Stellung am Oxazolidinring sitzt.

Die Oxazolidinone der gallbemeinen Formel (I) oder (II) lassen sich auf mehrstufigen Synthesewegen unter Verwendung von literaturbekannten Einzelreaktionen gewinnen. Auf die allgemeine Darstellungsweisen wird im Experimentellen Teil näher eingegangen.

Die genannten Oxazolidinone sind als Komponenten für Flüssigkristallmischungen geeignet. Dabei enthalten die LC-Mischungen vorzugsweise 0,01 bis 60, insbesondere 0,1 bis 20 Gew.-%, besonders bevorzugt 0,1 bis 5 Gew.-% an Oxazolidinonen. Die anderen Bestandteile werden vorzugsweise ausgewählt aus den bekannten Verbindungen mit nematischen, cholesterischen und/oder smektischen Phasen; dazu gehören beispielsweise Schiffsche Basen, Biphenyle, Terphenyle, Phenylcyclohexane, Cyclohexylbiphenyle, N, S oder O-haltige Heterocyclen, z.B. Pyrimidine, Zimtsäureester, Cholesterinester oder verschieden überbrückte, terminalpolar mehrkernige Ester von p-Alkylbenzoesäuren. Im allgemeinen liegen die im Handel erhältlichen Flüssigkristallmischungen bereits vor der Zugabe der optisch aktiven Verbindung(en) als Gemische verschiedener Komponenten vor, von denen mindestens eine mesogen ist, d.h. als Verbindung, in derivatisierter Form oder im Gemisch mit anderen Komponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwarten läßt.

Das hohe Verdrillungsvermögen der erfindungsgemäßen Verbindungen führt bereits in nematischen Phasen bei den eher "klassischen" Displaytechnologien zu vorteilhaften Einsatzmöglichkeiten. Hierbei steht jedoch häufig nicht die Kompensation, sondern die Erzielung einer Verdrillung durch eine möglichst geringe Zugabemenge an chiralem Dotierstoff im Vordergrund. Dies gilt sowohl für die TN ("twisted-nematic")-Technologie [siehe M. Schadt et al, Appl. Phys. Lett 18, 127 (1971)] als auch für das sogenannte White-Taylor-Display [D. L. White et al., J. Appl. Phys. 45, 4718 (1974)] oder das SBE/STN ("super birefringence-effect"/"super-twisted-nematic")-Display [T. J. Scheffer et al., Appl. Phys. Lett. 45, 1021 (1984)] und seine verschiedenen Modifikationen wie das OMI ("optical mode interference") Display [M. Schadt et al., Appl.

Phys. Lett. 50, 236 (1987)].

Auch in der $S_C$-Phase induzieren die erfindungsgemäßen Verbindungen eine Helix, so daß dieser Effekt benutzt werden kann, eine Verdrillung in der $S_C^*$ -Phase zu kompensieren oder auf einen bestimmten Wert einzustellen, was für den praktischen Einsatz vorteilhaft ist [z.B. T. Tsuchiga et al., Jpn. J. Appl. Phys. 25, L-27 (1986)].

Die Flüssigkristallmischungen können beispielsweise in elektrooptischen Schalt- und Anzeigevorrichtungen eingesetzt werden, die darüber hinaus u.a. folgende Komponenten enthalten: zwei Elektroden, zwei Trägerplatten sowie mindestens eine Orientierungsschicht. Allgemein wird der Aufbau von FLC-Displays in EP-B 0 032 362 beschrieben.

Die Erfindung wird durch die nachfolgenden Beispiele näher erläutert.

Beispiele

Allgemeiner Syntheseweg für die Oxazolidinone der allgemeinen Formel (I)

Ausgehend von einer (vorzugsweise natürlichen) (chiralen) Aminosäure bzw. einem Aminoalkohol wird das Oxazolidinon-System in einer mehrstufigen Synthese aufgebaut. Die Ankopplung der mesogenen Gruppierung kann in einem Reaktionsschritt oder durch stufenweisen Aufbau des Mesogens am Oxazolidinon erfolgen.

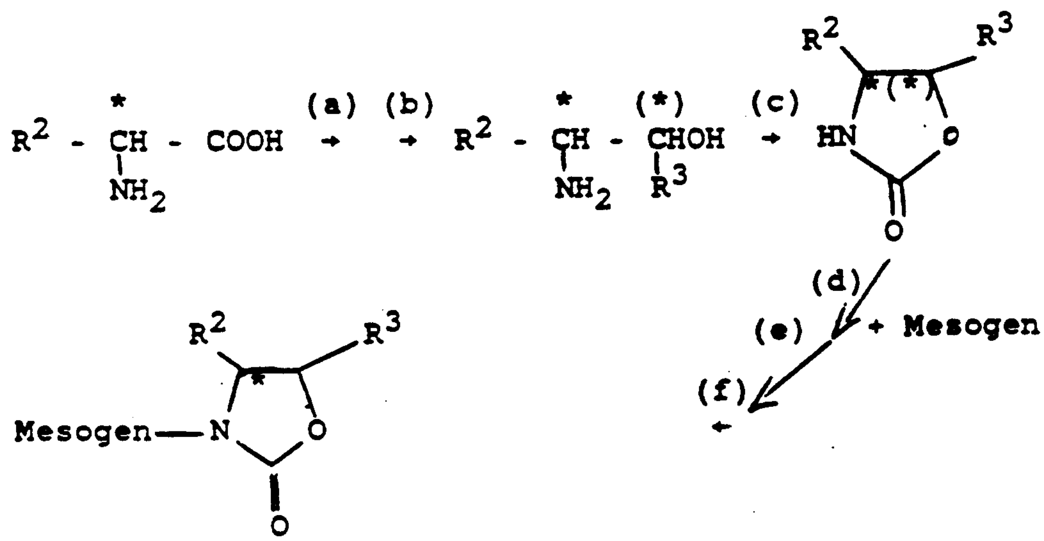


a) Reduktion (z.B. Lithiumaluminiumhydrid)

b) Einbringen des Restes "$R^3$", z.B. Oxidation mit PCC (Pyridiniumchlorochromat) danach Grignard-Reaktion

c) Cyclisierung mittels Phosgensynthon

d, e, f) Ankopplung bzw. schrittweiser Aufbau der mesogenen Seitenkette $(R^1(-A^1)_j(-M^1)_k(-A^2)_l(-M^2)_m(-A^3)_n$-X-).

Als Beispiel sei schematisch die Synthese und Ankopplung eines Biphenyl-Mesogens an das Oxazolidinon beschrieben:

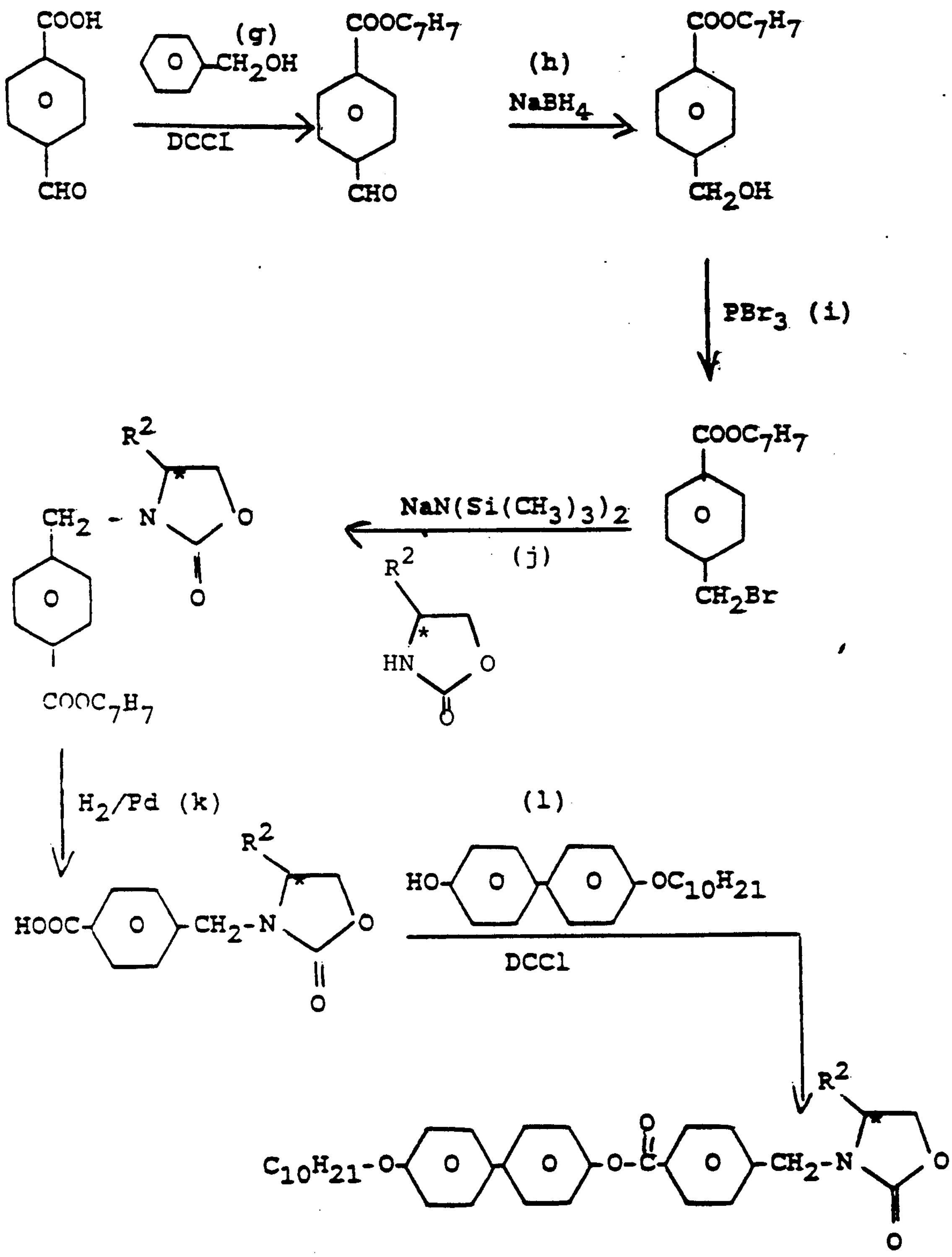


g) Veresterung z.B. mit Dicyclohexylcarbodiimid (DCCI)
h) Reduktion mit z.B. Natriumborhydrid
i) Überführung ins Bromid mit $PBr_3$
j) Ankondensieren an den Oxazolidinonring
k) Hydrierung des Esters z.B mit Pd/Kohle
l) siehe g).

Die Oxazolidinone der allgemeinen Formel (II) lassen sich beispielsweise nach folgendem Schema synthetisieren:

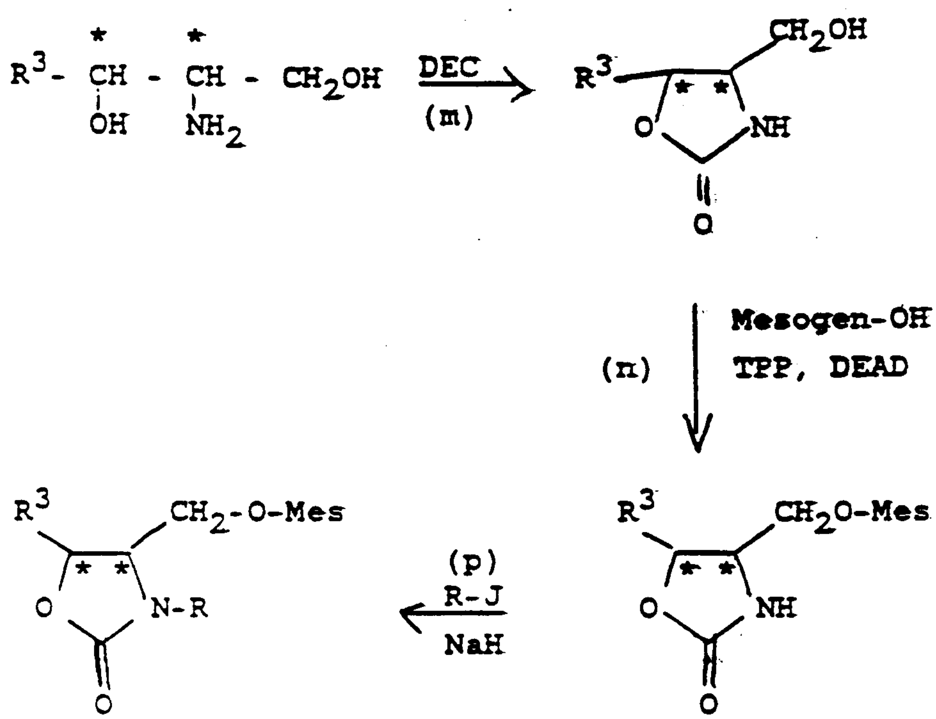


m) Kondensation mittels z.B. Diethylcarbonat (DEC)

n) Veretherung mit mesogenem Alkohol z.B. mit Triphenylphosphin (TPP), Diethylazodicarboxylat (DEAD)

p) N-Alkylierung mittels z.B. Alkyljodid/Natriumhydrid

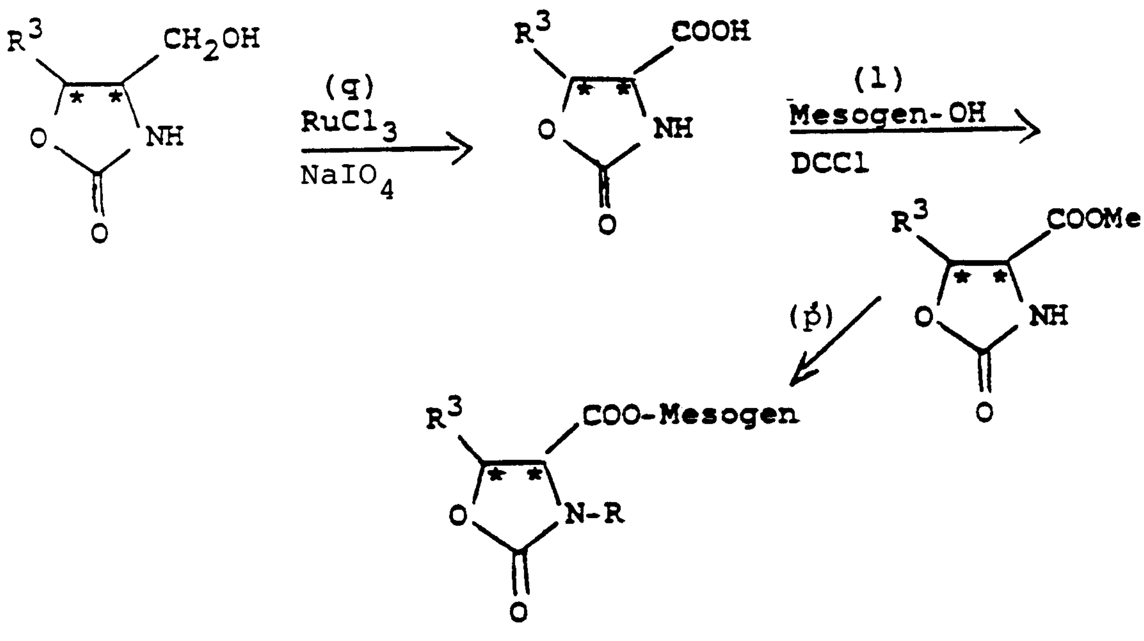


q) Oxidation z.B. mit Natriumperjodat/Ruthenium-(III)-chlorid-Trihydrat

Die nachfolgenden Beispielverbindungen wurden nach den oben skizzierten Schemata unter Anwendung literaturbekannter Einzelreaktionen hergestellt.

Synthese der Ausgangsstoffe:

A. (2R)-(-)-2-Amino-2-phenyl-ethanol

Zu einer Suspension von 3,66 g (96 mmol) Lithumaluminiumhydrid (LAH) in 130 ml abs. THF werden portionsweise 10 g (66 mmol) D-Phenylglycin gegeben. Nach üblicher Aufarbeitung und Destillation (Badtemperatur: 90 °C bei 0,8 Torr) resultiert eine farblose, zähe Flüssigkeit.
Ausbeute: 7.7 g (56 mmol) = 85 %
$[\alpha]_D^{24}$ = -50,0 ° (c = 3,22, $CHCl_3$)
Summenformel: $C_8H_{11}NO$ (M = 137,2)

B. (4R)-(-)-4-Phenyl-2-oxazolidinon

7,54 g (55 mmol) (2R)-(-)-2-Amino-2-phenyl-ethanol werden in 8 ml (61 mmol) Diethylcarbonat gelöst, mit 770 mg (5,5 mmol) $K_2CO_3$ versetzt und Ethanol über eine Kolonne abdestilliert. Das Rohprodukt wird aus Ether/Petrolether umkristallisiert.
Ausbeute: 6,2 g (38 mmol) = 69 % (farblose Kristalle) Smp: 129 °C

C. (4R)-(-)-3-(4-Benzyloxycarbonyl-phenylmethyl)-4-phenyl-2-oxazolidinon

490 mg (3 mmol) (4R)-(-)-4-Phneyl-2-oxazolidinon werden in 9 ml abs. THF gelöst, zuerst mit 3,3 ml Natriumbistrimethylsilylamid (1 M in THF) und dann mit 1050 mg (3,45 mmol) 4-Brommethyl-benzoesäurebenzylester, gelöst in 2 ml abs. THF, versetzt. Das Rohprodukt wird chromatograpisch (Laufmittel: $CH_2Cl_2$) gereinigt.
Ausbeute: 770 mg (2 mmol) = 66 % (farbloses Öl)
$[\alpha]_D^{23}$ = -85,9 ° (c = 1,70)

D. (4R)-(-)-3-(4-Carboxy-phenylmethyl)-4-phenyl-2-oxazolidinon

710 mg (1,8 mmol) (4R)-(-)-3-(4-Benzyloxycarbonylphenylmethyl)-4-phenyl-2-oxazolidinon werden in 18 ml abs. Ethanol gelöst, mit 70 mg Pd/C 10 % versetzt und hydriert.
Ausbeute: 540 mg (1,8 mmol) = 99 %
$[\alpha]_D^{24}$ = -102,5 ° (c = 2,85; $CHCl_3$)
IR: 3200-2300 (COOH); 1750, 1700 (C=O); 1615 (C=C); 1410; 1110

E. (2S)-(+)-2-Amino-1-propanol

Zu einer Suspension von 5,55 g (146 mmol) Lithiumaluminiumhydrid in 200 ml abs. THF werden portionsweise 8,9 g (100 mmol) L-Alanin eingetragen. Übliche Aufarbeitung und Destillation (Badtemperatur: 70 °C bei 4 Torr) liefert ein farbloses Öl.
Ausbeute: 3,37 g (45 mmol) = 45 %
$[\alpha]_D^{23}$ = +19,6 ° (c = 1,20; MeOH)
Summenformel: $C_3H_9NO$ (M = 75,1)

F. (4S)-(+)-4-Methyl-2-oxazolidinon

3,2 g (42,5 mmol) (2S)-(+)-2-Amino-1-propanol werden in 5,6 g (47 mmol) Diethylcarbonat gelöst, mit 600 mg (4,3 mmol) $K_2CO_3$ versetzt und über eine Kolonne EtOH abdestilliert. Destillation (Badtemperatur: 160 °C bei 0,1 Torr) liefert farblose Kristalle.
Ausbeute: 2,60 g (25,5 mmol) = 60 % Smp.: 66 °C
$[\alpha]_D^{23}$ = +7,8 ° (c = 3,63; $CHCl_3$)
IR: 3460, 3270 (NH); 1755 (C=O); 1460; 1040; 945

G.

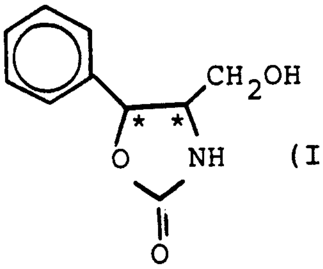 (I)

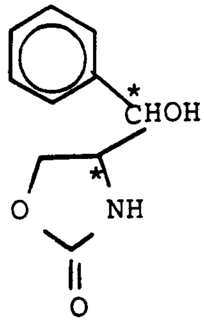 (II)

In einem 50 ml Kolben werden 8,35 g (50 mmol) 1-Amino-3-phenyl-1,3-propandiol, 7,2 ml (55 mmol) Diethylcarbonat und 700 mg (5 mmol) $K_2CO_3$ gegeben. Es wird eine Vigreux-Kolonne mit Destillationsbrükke aufgesetzt. Die Suspension wird ca. 4 h auf 130° C erhitzt, bis 2 Equivalente Ethanol abdestilliert sind. Die abgekühlte Mischung wird in Methanol aufgenommen und das $K_2CO_3$ über Cellite abfiltriet. Nach dem Einengen wird das Rohprodukt chromatographisch gereinigt (Kieselgel/Ether/Methanol). Nach NMR liegen die Verbindungen (I) und (II) im Verhältnis 9:1 im kristallinen Produkt vor. Eine weitere Trennung ist nicht erforderlich, da bei den nachfolgenden Reaktionen nur (I) umgesetzt wird.
Ausbeute I + II: 9,26g = 96 % Schmelzpunkt 55° C

H. Oxidation von G. zur Säure

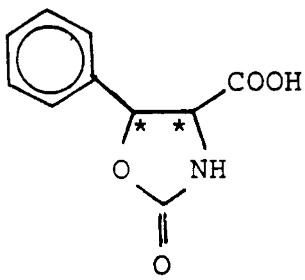

In der angegebenen Reihenfolge werden 17,5 ml Tetrachlormethan, 17,5 ml $CH_3CN$, 27,3 ml $H_2O$, 2,0 g (10,5 mmol) (I), 6,7 g (31,5 mmol) $NaIO_4$ und 108 mg $RuCl_3 \cdot 3H_2O$ zusammengegeben und über Nacht bei 20° C gerührt. Der Ansatz wird eingeengt, in 70 ml ges. NaCl-Lsg. und 70 ml $CH_2Cl_2$ aufgenommen. Man trennt die Phasen, extrahiert die wäßrige zweimal mit $CH_2Cl_2$, trocknet über $MgSO_4$ und entfernt das Lösungsmittel in Vakuum. Das Rohprodukt wird mittels Säulenchromatographie gereinigt ($CH_2Cl_2/Et_2O$)
Ausbeute 650 mg (45 %)

Beispiel 1

(4S)-3-[4-(4'-Decyloxy-biphenyl-4-oxycarbonyl)benzyl]-4-isopropyl-2-oxazolidinon

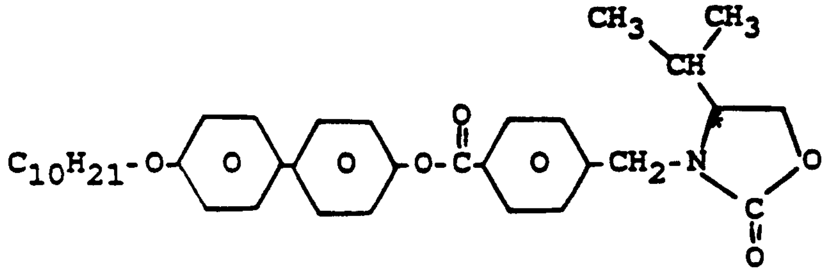

Schmelzpunkt : 148 °C
$[\alpha]_D^{23}$ : -6,9° (c = 1.45, $CHCl_3$)

Beispiel 2

(4R)-3-[4-(4'-Decyloxy-biphenyl-4-oxycarbonyl)benzyl]-4-

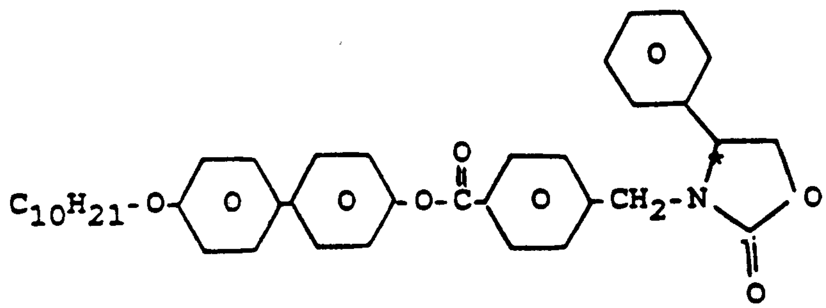

phenyl-2-oxazolidinon Schmelzpunkt : 158 °C
$[\alpha]_D^{23}$ : -90,2° (c = 3.6, $CHCl_3$)
505 mg (1,07 mmol) (4R)-(-)-3-(4-Carboxy-phenylmethyl)-4-phenyl-2-oxazolidinon und 555 mg (1,7 mmol) 4-Decyloxy-4'-hydroxy-biphenyl und 31 mg DMAP werden in 17 ml abs. $CH_2Cl_2$ gelöst und mit 390 mg (1,7 mmol) DCCI versetzt. Die Reinigung des Rohproduktes erfolgt chromatographisch.
Ausbeute: 800 mg (1,3 mmol) = 78 % (farbloser Feststoff)
IR: 2930, 2860 (CH); 1740 (C=O); 1610, 1500 (C=C); 1410; 1270; 1080

Beispiel 3

(4S)-3-[4-(4'-Decyloxy-biphenyl-4-oxycarbonyl)benzyl]-4-methyl-2-oxazolidinon

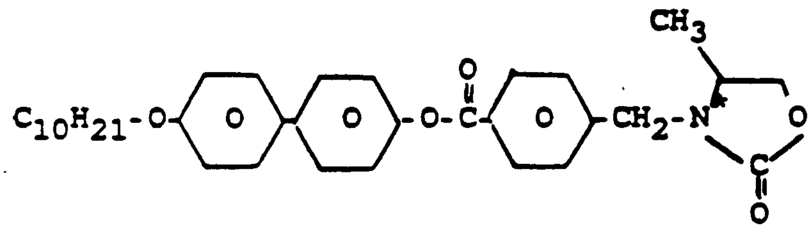

Schmelzpunkt : 122 °C Phasenfolge: X 122 Sc* 134 I
$[\alpha]_D^{23}$ : -15,5° (c = 1.55, $CHCl_3$)

Beispiel 4

(5S)-3-[4-(4'-Decyloxy-biphenyl-4-oxycarbonyl)benzyl]-5-methyl-2-oxazolidinon

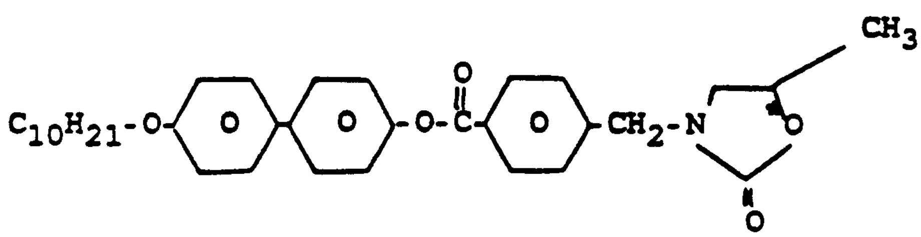

Schmelzpunkt : 183 °C

$[\alpha]_D^{23}$ : -14,7° (c = 1.7, $CHCl_3$)

Beispiel 5

(4S,5S)-4-[4-(2-Octyl-pyrimidin-5-yl)phenyloxymethyl]-3-methyl-5-phenyl-2-oxazolidinon

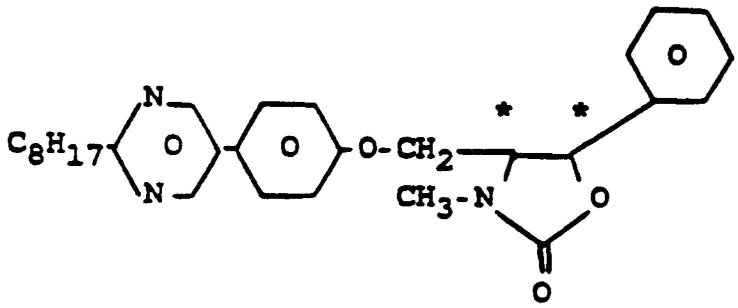

(5)

Schemlzpunkt : 114° C
$^1$HNMR: δ = 0,87 (t, 3H); 1,21 - 1,40 (m, 12H); 1,64 (2H); 2,60 (t, 7,5, 2H); 3,00 (S, 3H); 3,92 (t, 1H); 4,23 (dd, 1H); 4,26 (dd, 1H); 5,36 (d, 1H); 7,02 - 8,39 (9H, 4H); 7,35 - 7,45 (m, 5H); 8,58 (s, 2H)

Beispiel 6

4-[4-(2-Decyl-pyrimidin-5-yl)phenyloxymethyl]-5-phenyl-2-oxazolidinon

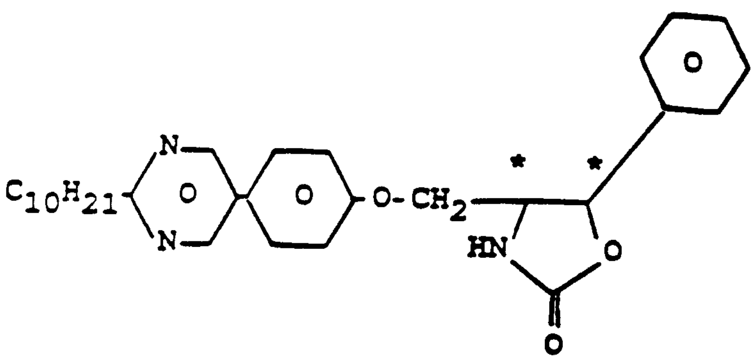

(6)

Schmelzpunkt : 131° C
$^1$HNMR: δ = 0,87 8 (t, 3H); 1,20 - 1,40 (m, 16Hz); 1,64 (q, 7,5Hz, 2H); 2,60 (t, 7,5Hz); 4,13 - 4,11 (m, 3H); 5,42 (dd, 1H); 5,80 - 5,87 (m, 1H); 6,98; 8,37 (8,5Hz); 7,37 - 7,47 (m, 5H); 8,59 (S, 2H)

In einem Kolben werden 538 mg (3 mmol) des Oxazolidinylmethylalkohols, 786 mg (3 mmol) Triphenylphosphin und 984 mg (3 mmol) des Pyrimidinderivates in 50 ml abs. THF gelöst und mit 525 mg (3 mmol) DEAD versetzt. Die Lösung wird 1 Tag bei 20° C gerührt. Nach Abziehen des THF wird das Rohprodukt zweimal säulenchromatographisch gereinigt (Kieselgel/Methylenchlorid/Ether)
Ausbeute: 750 mg (50 %) Schmelzpunkt 131° C

Beispiel 7

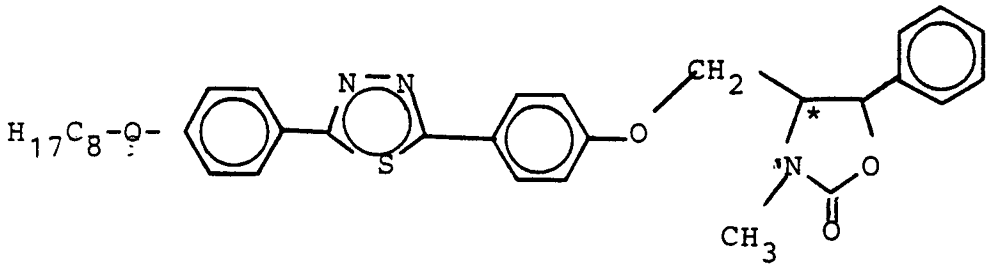

Schmelzpunkt: 158 ° C

$^1$HNMR: δ = 0,89 (t, 7Hz, 3H); 1,25 - 1,41 (m, 8H); 1,47 (quint); 1,81 (quint, 7Hz, 2H); 3,00 (S, 3H); 3,93 (dt, 1H); 4,02 (t, 7Hz, 2H); 4,22 (dd, 1H); 4,27 (dd, 1H); 5,36 (d, 6Hz, 1H); 6,98/7,92 (8,5Hz, 4H); 7,03/7,96 (8,5Hz, 4H); 7,37 - 7,46 (m, 5H)

Beispiel 8

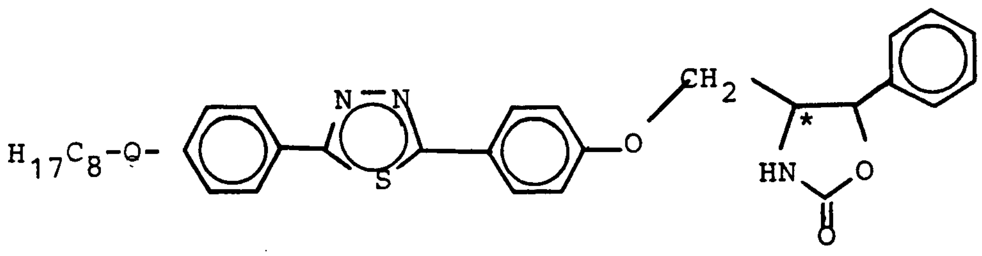

Schmelzpunt 203 ° C

$^1$HNMR: δ = 0,88 (t, 7Hz, 3H); 1,23 - 1,39 (m, 8H); 1,47 (quint, 7Hz); 1,81 (quint, 7Hz, 2H); 4,02 (t, 7Hz, 2H); 4,13 (td, 1H); 4,17 (dd, 1H); 4,20 (dd, 1H); 5,36 (d, 5Hz, 1H); 6,89/7,78 (9Hz, 4H); 6,94/7,92 (9Hz, 4H); 7,37 - 7,46 (m, 5H)

Beispiel 9

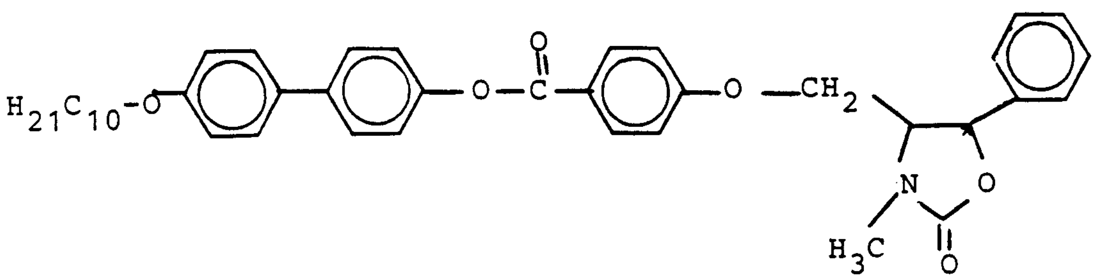

Schmelzpunkt 143 ° C

$^1$HNMR: δ = 0,89 (t, 7Hz, 3H); 1,24 - 1,42 (m, 12H); 1,47 (quintbr, 7Hz, ZM); 1,81 (quint, 7Hz, 2H); 3,01 (S, 3H); 3,94 (t, 6 und 4,5HZ, 1H); 4,00 (t, 7Hz, 2H); 4,25 (dd, 10 und 4,5Hz, 1H); 4,29 (dd, 10 und 4,5Hz, 1H); 5,37 (d, 6Hz, 1H); 6,97/7,51 (AA'BB', 8,5Hz, 4H); 7,04/8,21 (AA'BB', 8,5Hz, 4H); 7,37 - 7,47 (m, 5H)

Beispiel 10

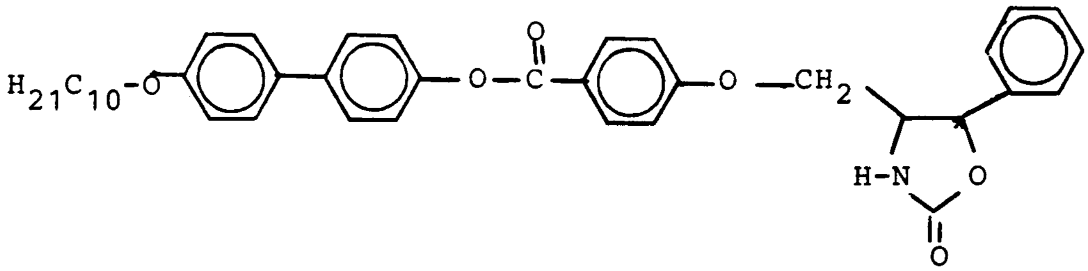

Schmelzpunkt 190 ° C
$^1$HNMR: δ = 0,89 (t, 7Hz, 3H); 1,19 - 1,35 (m, 12H); 1,46 (quintbr, 7Hz, 2H); 1,79 (quint, 7Hz, 2H); 4,03 (t, 7Hz, 2H); 4,36 (td, 5,5 und 5Hz); 4,43 (dd, 9,5 und 5,5Hz, 1H); 4,46 (dd, 9,5 und 5,5Hz, 1H); 5,73 (d, 5Hz, 1H); 7,21/7,70 (AA'BB', 9Hz, 4H); 7,21/8,33 (AA', BB', 9Hz); 7,51/7,78 (AA', BB', 9Hz, 4H); 7,60 - 7,68 (m, 5H)

Anwendungsbeispiele

Meßmethoden

Die spontane Polarisation (Ps) wird in einer 10 μ Zelle nach der Methode von Diamant et al. (Rev. Sci. Instr., 28, 30, 1957) gemessen. Bei einer Zellenschichtdicke von ca. 2 μm wird durch Orientierungsschichten eine einheitliche planare Orientierung der Flüssigkristalle in der $S_C^*$ -Phase erreicht [siehe SSFLC-Technik, Clark et al, Appl. Phys. Lett. 36, 899 (1980)]. Zur Bestimmung von $\tau$ und $\theta$ wird die Meßzelle auf dem Drehtisch eines Polarisationsmikroskops zwischen gekreuztem Analysator und Polarisator befestigt. Der Schaltwinkel ($2\theta$) wird bei an der Meßzelle angelegtem statischen elektrischen Feld gemessen. Für positive und negative Polarität dieses Feldes wird die Meßzelle jeweils so lang gedreht bis minimaler Lichtdurchgang auftritt. Der Winkelunterschied zwischen den beiden so ermittelten Orientierungen ergibt den Schaltwinkel. Mit Hilfe einer Photodiode erfolgt die Bestimmung der Schaltzeit ($\tau$) indem die Anstiegszeit des Lichtsignals von 10 auf 90 % Signalhöhe gemessen wird. Die Schaltspannung beträgt ± 10 V/μm. Neben den Werten für $P_S$, $\tau$, und $2\theta$ ist der S*-Bereich der jeweiligen Mischung angegeben; die Werte in Klammern geben dabei die unterkühlbare untere Temperaturgrenze des $S_C$-Bereichs an.

Die Bestimmung des Pitches (Z) und der Verdrillungskraft (HTP) in der cholesterischen Phase erfolgte, wie z.B. bei P. Kassubek et al., Mol. Cryst. Lig. Cryst., Vol. 8, 305-314, 1969 beschrieben, in einer Keilzelle mit Orientierungsschicht durch Ausmessen der Versetzungslinien unter dem Polarisationsmikroskop.

Anwendungsbeispiel A1

a) Eine flüssigkristalline Mischung bestehend aus 6 Komponenten

| | |
|---|---|
| 5-Octyloxy-2-(4-butyloxy-phenyl)-pyrimidin | 26,1 Mol-% |
| 5-Octyloxy-2-(4-hexyloxy-phenyl)-pyrimidin | 24,8 Mol-% |
| 5-Octyloxy-2-(4-octyloxy-phenyl)-pyrimidin | 11,4 Mol-% |
| 5-Octyloxy-2-(4-decyloxy-phenyl)-pyrimidin trans-4-Pentyl-cyclohexancarbonsäure- | 20,9 Mol-% |
| [4-(5-decyl-pyrimidin-2-yl)]-phenylester (5S)-3-[4-(4'-Decyloxy-biphenyl-4- | 14,8 Mol-% |
| oxycarbonyl]benzyl-5-methyl-2-oxazolindinon | 2 Mol-% |

zeigt folgende flüssigkristalline Phasenbereiche:
X 9 $S_C$* 81,3 $S_{A^*}$ 91 N* 102,5 I
Die HTP des Dotierstoffes ist in folgender Tabelle wiedergegeben.

| | | |
|---|---|---|
| HTP [$\mu m^{-1}$] | 1,45 | 1,96 |
| $\tau$ [ ° C] | 92 | 95 |

b) im Vergleich dazu weist die, in DE 3831226.3 beanspruchte flüssigkristalline Mischung, die sich von

der obgengenannten Mischung nur dadurch unterscheidet, daß sie keinen Dotierstoff enthält, folgende Phasenbereiche auf:
X 9 $S_C$ 84 $S_A$ 93 N 105 I

Anwendungsbeispiel A2

a) Eine flüssigkristalline Mischung bestehend aus 6 Komponenten

| | |
|---|---|
| 5-Octyloxy-2-(4-butyloxy-phenyl)-pyrimidin<br>5-Octyloxy-2-(4-hexyloxy-phenyl)-pyrimidin<br>5-Octyloxy-2-(4-octyloxy-phenyl)-pyrimidin<br>5-Octyloxy-2-(4-decyloxy-phenyl)-pyrimidin trans-4-Pentyl-cyclohexancarbonsäure-<br>[4-(5-decyl-pyrimidin-2-yl)]-phenylester (4R)-3-[4-(4'-Decyloxy-biphenyl-4-<br>oxycarbonyl]benzyl-4-phenyl-2-oxazolidinon | 26,1 Mol-%<br>24,8 Mol-%<br>11,4 Mol-%<br>20,9 Mol-%<br>14,8 Mol-%<br>2 Mol-% |

zeigt folgende flüssigkristalline Phasenbereiche:
X 9 $S_C^*$ 76 $S_{A^*}$ 89 N* 101 I
Die HTP des Dotierstoffes ist in folgender Tabelle wiedergegeben.

| HTP [$\mu m^{-1}$] | 12,1 | 13,5 | 14,5 | 15,7 | 17,2 |
|---|---|---|---|---|---|
| $\tau$ [°C] | 89 | 91 | 93 | 95 | 97 |

Im Vergleich dazu weisen andere Mischungen, die sich von der obengenannten dadurch unterscheiden, daß sie andere Dotierstoffe (ebenfalls zu 2 Mol-% in der Geamtmischung) enthalten, folgendes in Tabelle 1 genanntes Verdrillungsvermögen auf:

Tabelle 1 Die HTP-Werte von Vergleichsmischungen verschiedener Dotierstoffe in der Grundmischung Beispiel A1 wurden bei einer Temperatur von 95°C ermittelt:

| Vergleichs-beispiel | Struktur | | HTP [µm$^{-1}$] |
|---|---|---|---|
| 2b | 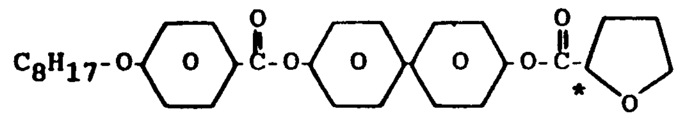 | (R) | + 0,1 |
| 2c | 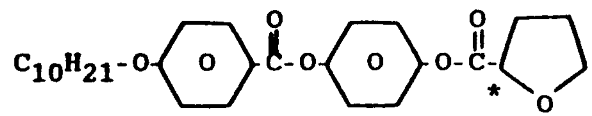 | (R) | + 1,1 |
| 2d | 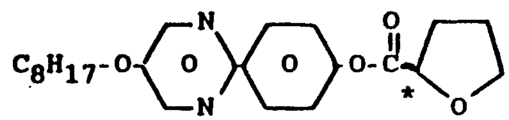 | (R) | + 2 |
| 2e | 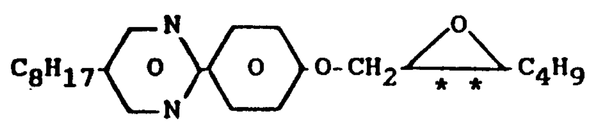 | (2S,3S) | - 1,5 |

**Fortsetzung Tabelle 1**

| Vergleichsbeispiel | Struktur | | HTP [$\mu m^{-1}$] |
|---|---|---|---|
| 2f | 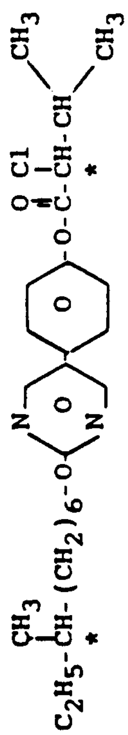 | (2S,3S) | - 0,15 |
| 2g | 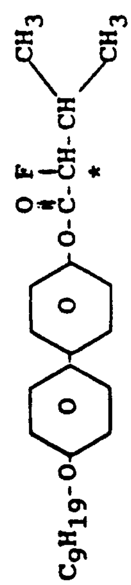 | (S) | - 1,7 |
| 2h | 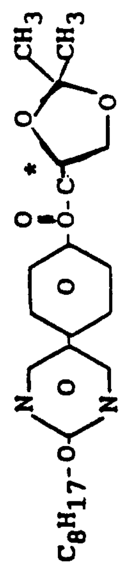 | (R) | - 0,7 |

Anhand der Tabelle 1 erkennt man, daß der erfindungsgemäße Dotierstoff in der Basis-Mischung aus A1 eine überraschend große HTP aufweist und daher in besonderem Maße zur Kompensation des Pitches anderer Dotierstoffe geeignet ist.

Anwendungsbeispiel A3

a) Eine flüssigkristalline Mischung bestehend aus 6 Komponenten

| | |
|---|---|
| 5-Octyloxy-2-(4-butyloxy-phenyl)-pyrimidin | 26,1 Mol-% |
| 5-Octyloxy-2-(4-hexyloxy-phenyl)-pyrimidin | 24,8 Mol-% |
| 5-Octyloxy-2-(4-octyloxy-phenyl)-pyrimidin | 11,4 Mol-% |
| 5-Octyloxy-2-(4-decyloxy-phenyl)-pyrimidin | 20,9 Mol-% |
| trans-4-Pentyl-cyclohexancarbonsäure-[4-(5-decyl-pyrimidin-2-yl)]-phenylester | 14,8 Mol-% |
| (4S)-3-[4-(4'-Decyloxy-biphenyl-4-oxycarbonyl]benzyl-4-isopropyl-2-oxazolidinon | 2 Mol-% |

zeigt folgende flüssigkristalline Phasenbereiche:
X 9 $S_C$* 79 $S_{A*}$ 90 N* 102.5 I
Die HTP des Dotierstoffes ist in folgender Tabelle wiedergegeben.

| HTP [$\mu m^{-1}$] | 5,7 | 6,2 | 6,6 | 7,3 |
|---|---|---|---|---|
| $\tau$ [°C] | 90 | 93 | 96 | 99 |

Auch dieser Dotierstoff weist eine HTP auf, die wesentlich höher liegt als bei den in der Tabelle 1 aufgeführten Vergleichsdotierstoffen. Somit ist auch dieser Dotierstoff sehr zur Kompensation eines Pitches geeignet.

## Patentansprüche

**1.** Chirales Oxazolidinon gemäß den allgemeinen Formeln (I) oder (II)

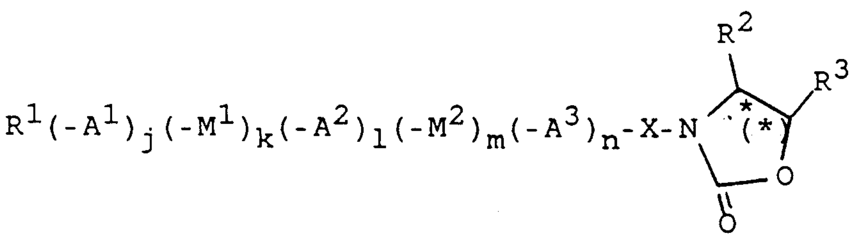

(I)

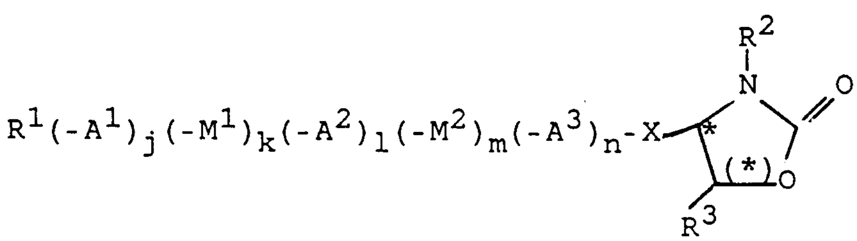

(II)

wobei die Symbole folgende Bedeutung haben:

* chirales Zentrum,

(*) gegebenenfalls chirales Zentrum,

$R^1$ ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, eine oder mehrere nicht benachbarte $-CH_2$-Gruppen durch -O-, -S-, -CO-, -O-CO- und/oder -CO-O- ersetzt sein können, und wobei ein oder mehrere H durch F, Cl, Br oder CN ersetzt sein können,

$-R^2$, $-R^3$ unabhängig voneinander -H, ein Alkyl mit 1 bis 12 C-Atomen, bei dem eine $-CH_2$-Gruppe auch durch -O-oder -S- ersetzt sein kann, und bei dem die terminale $CH_3$-Gruppe auch durch $CH_2OR^4$,

$CH_2SR^4$ oder $CH_2CONH_2$ ersetzt sein kann wobei $R^4$ gleich Alkyl 1 bis 10 C-Atomen ist, -Phenyl oder -Cyclohexyl, die auch durch $-OR^4$, -F oder -J substituiert sein können,
j und l null, 1 oder 2,
k und m null oder 1,
n null, 1 oder 2,

mit folgender Maßgabe: wenn j und/oder l = null sind, ist k = null; wenn n = null ist, ist m = null; die Summe j + l + n ist minimal 1 und maximal 3,

$-A^1$, $-A^2$

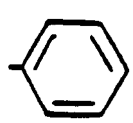 , 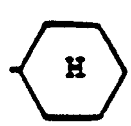 , 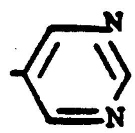 , 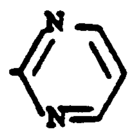 , 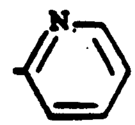 ,

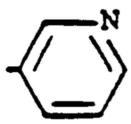 , 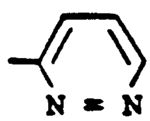 , 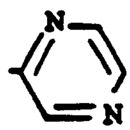 , 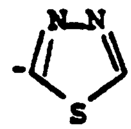 , 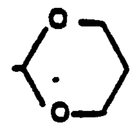 ,

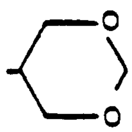 , 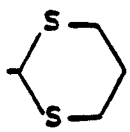 , 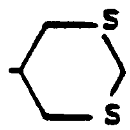 , 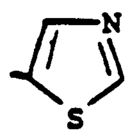 , 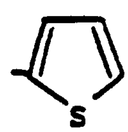 ,

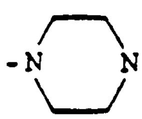 , 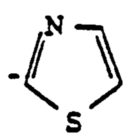

$-A^3$

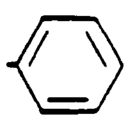 , 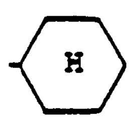 , 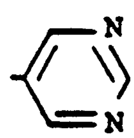 , 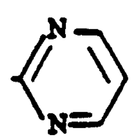 , 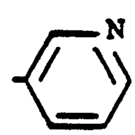 ,

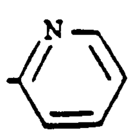 , 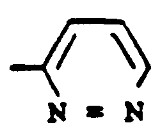 .

$-M^1$, $-M^2$ -CO-O, -O-CO, $-CH_2CH_2$, -CH=CH, $-CH_2O$, $-OCH_2$, -X- $-O-CH_2$, $-CH_2-$,

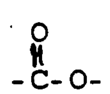 , 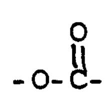 ,

$-CH_2-O-$, $-C_2H_4-$.

2. Oxazolidinon der allgemeinen Formel (I) oder (II) gemäß Anspruch 1, dadurch gekennzeichnet, daß

$R^1$ ein geradkettiger oder verzweigter Alkyl- oder Alkenylrest mit 4 bis 14 C-Atomen ist, der ein asymmetrisches C-Atom enthalten kann, und wobei eine $-CH_2$-Gruppe durch -O-, -CO- oder -CO-O- ersetzt sein kann, und

$-R^2$, $-R^3$ - H , $-CH_3$,

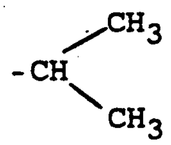 , 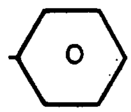 , 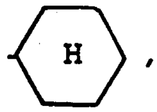 ,

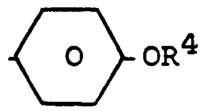

j, k, l, m, n null oder 1 bedeuten.

3. Oxazolidinon der allgemeinen Formel (I) oder (II) gemäß Anspruch 1, dadurch gekennzeichnet, daß

$R^1$ ein geradkettiger oder verzweigter Alkyl- oder Alkenylrest mit 6 bis 12 C-Atomen ist, der ein asymmetrisches C-Atom enthalten kann und wobei eine $CH_2$-Gruppe durch -O-, -S-, -CO-, -O-CO- oder -COO-ersetzt sein kann, und

die Gruppierung $(-A^1)_j(-M^1)_k(-A^2)_l(-M^2)_m(-A^3)_n$-folgende Bedeutung hat:

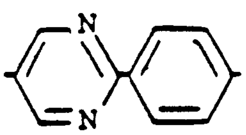 , 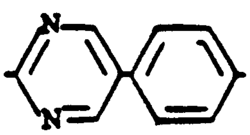 , 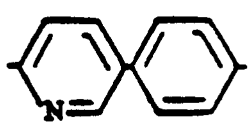 ,

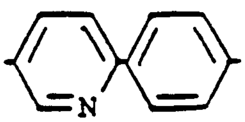 ,

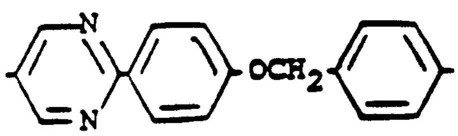 , 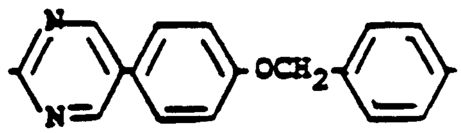 ,

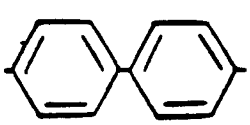 , 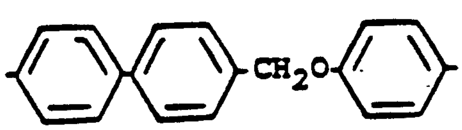 ,

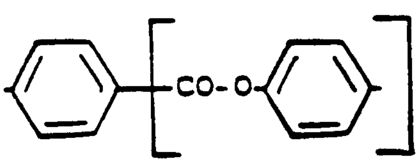 1 oder 2 , 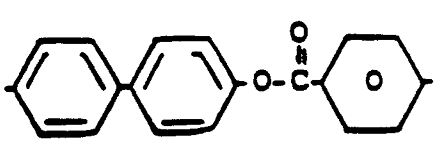

4. Flüssigkristallmischung bestehend aus mindestens zwei Komponenten, dadurch gekennzeichnet, daß sie mindestens ein chirales Oxazolidinon gemäß Anspruch 1 enthält.

5. Ferroelektrische Flüssigkristallmischung bestehend aus mindestens zwei Komponenten, dadurch gekennzeichnet, daß sie als Dotierstoff ein Oxazolidinon gemäß Anspruch 1 enthält.

6. Ferroelektrische Flüssigkristallmischung bestehend aus mindestens zwei Komponenten, dadurch gekennzeichnet, daß sie als Dotierstoff ein Oxazolidinon gemäß Anspruch 2 enthält.

7. Ferroelektrische Flüssigkristallmischung bestehend aus mindestens zwei Komponenten, dadurch gekennzeichnet, daß sie als Dotierstoff ein Oxazolidinon gemäß Anspruch 3 enthält.

8. Ferroelektrische Flüssigkristallmischung bestehend aus zwei Komponenten, dadurch gekennzeichnet, daß sie 0,1 bis 20 Gew.-% eines Oxazolidinons gemäß Anspruch 1 enthält.

9. Verwendung von chiralen Oxazolidinonen der allgemeinen Formel (I) oder (II) gemäß Anspruch 1 als Dotierstoff in Flüssigkristallmischungen.

10. Verwendung gemäß Anspruch 8, dadurch gekennzeichnet, daß die Flüssigkristallmischung eine smektische Phase besitzt.

11. Elektrooptische Schalt- und Anzeigevorrichtung enthaltend eine ferroelektrische Flüssigkristallmischung, zwei Elektroden, zwei Trägerplatten sowie mindestens eine Orientierungsschicht, dadurch gekennzeichnet, daß sie eine ferroelektrische Flüssigkristallmischung enthält, die als eine Komponente ein Oxazolidinon der Formel (I) oder (II) nach Anspruch 1 enthält.

12. Verfahren zur Herstellung von chiralen Oxazolidinonen der Formel (I) gemäß Anspruch 1.

13. Verfahren zur Herstellung von chiralen Oxazolidinonen der Formel (II) gemäß Anspruch 1.

Europäisches Patentamt

# EUROPÄISCHER RECHERCHENBERICHT

Nummer der Anmeldung

**EP 91 11 2046**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 306 919 (DAISO CO.)(15-03-1989)<br>* Insgesamt *<br>– – – | 1 | C 07 D 413/12<br>C 09 K 19/34<br>G 02 F 1/13<br>C 07 D 417/12<br>C 07 D 263/20 |
| A | EP-A-0 361 272 (HOECHST AG)(04-04-1990)<br>* Insgesamt *<br>– – – | 1 | |
| P,X | DATABASE WPIL, Accession Nr. 91-233825 [32], Derwent Publications Ltd, London, GB;<br>& JP-A-3 151 371 (DAINIPPON)<br>* Insgesamt *<br>– – – – – | 1 | |
| | | | **RECHERCHIERTE SACHGEBIETE (Int. Cl.5)** |
| | | | C 09 K 19/00<br>G 02 F 1/00<br>C 07 D 417/00<br>C 07 D 413/00<br>C 07 D 263/00 |

Der vorliegende Recherchenbericht wurde für alle Patentansprüche erstellt

| Recherchenort | Abschlußdatum der Recherche | Prüfer |
|---|---|---|
| Den Haag | 12 November 91 | OUSSET J-B. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument